# EUROPÄISCHE PATENTSCHRIFT

(11) **EP 0 923 517 B1**
(45) Veröffentlichungstag und Bekanntmachung des Hinweises auf die Patenterteilung: **13.06.2001**
(21) Anmeldenummer: 97944767.9
(22) Anmeldetag: 18.08.1997
(51) Int. Cl.: C07C 29/36, C07C 33/02, C07C 67/08, C07C 69/80

(54) **VERFAHREN ZUR TELOMERISIERUNG VON DIENEN**
PROCESS FOR TELOMERIZING DIENES
PROCEDE DE TELOMERISATION DE DIENES

(30) Priorität: 27.08.1996 DE 19634469
(43) Veröffentlichungstag der Anmeldung: 23.06.1999
(73) Patentinhaber: Celanese Chemicals Europe GmbH, 60439 Frankfurt am Main (DE)
(72) Erfinder: TAFESH, Ahmed, 24660 Acre (IL); BELLER, Matthias, D-85737 Ismaning (DE); KRAUSE, Jochen, D-60594 Frankfurt am Main (DE)
(86) Internationale Anmeldenummer: EP9704500
(87) Internationale Veröffentlichungsnummer: WO9808794

(56) Entgegenhaltungen:
- EP-A- 0 436 226
- EP-A- 0 571 819
- WO-A-95/30636
- GB-A- 2 093 025
- US-A- 4 142 060
- US-A- 4 356 333
- US-A- 5 345 007
- DATABASE WPI Section Ch, Week 9401 Derwent Publications Ltd., London, GB; Class A60, AN 94-005121 XP002051998 & SU 1 781 206 A (AS AZERB INORG PHYS CHEM INST) , 15.Dezember 1992

## Beschreibung

Die vorliegende Erfindung betrifft ein Verfahren zur Telomerisierung von Dienen. Insbesondere betrifft die vorliegende Erfindung ein Verfahren, gemäß dem das Dien in Gegenwart eines zweizähnigen Phosphinliganden und einer Palladiumverbindung zu dem entsprechenden Dimeren umgesetzt wird. Mit dem erfindungsgemäßen Verfahren kann das erwünschte Dimere in großer Ausbeute und hoher Selektivität erhalten werden.

### Stand der Technik

Über die Telomerisierung von ethylenisch ungesättigten Verbindungen wie z.B. Dienen lassen sich niedermolekulare Oligomere dieser Verbindungen erhalten, die wertvolle Ausgangsstoffe für die Synthese darstellen.

Beispielsweise lassen sich über die Telomerisierung von Dienen wie Butadien die entsprechenden Dimere herstellen. Insbesondere Butadien hat große Aufmerksamkeit gefunden, da es leicht mit geringen Kosten erhältlich ist und für viele Zwecke verwendet werden kann.

So ist bekannt, daß sich Butadien mit Alkoholen (Takahashi, S. Shibano, T. Hagihara, N.; Bull. Chem. Soc. Japan. 1968, 41, 454, (b) Tetrahedron Letter. 1967, 2451), Phenol (Smutny, E.J.; J. Am. Chem. Soc. 1967, 89, 6793), Carbonsäuren (Manyik, R.M. Walker, W.E. Atkins, K.E.; Chemical Communication, 1971, 330), Wasser (Tsuji, J. Takahashi, M.; J. Molec. Catalysis. 1981, 10, 107), Ammoniak (Tsuji, J. Mori, Y.; Tetrahedron. 1972, 28, 3721) und Kohlenstoffmonoxid (Kohle, J.F. Slaugh, L.H. Nakamaye, K.L.; J. Am. Chem. Soc. 1969, 91, 5904) zu den entsprechenden Octadienylethern, -estern, -alkoholen, -aminen sowie - carbonsäuren umsetzen läßt.

Diese Telomerisierung genannten Umsetzungen erfolgen üblicherweise in Gegenwart eines Katalysatorsystems, das eine Übergangsmetallverbindung und einen Komplexliganden enthält. Bekannte Übergangsmetallverbindungen sind Palladiumverbindungen und bekannte Komplexliganden sind einzähnige Phosphinliganden.

Beispielsweise stellt die Umsetzung von Butadien mit Wasser in Gegenwart eines Palladiumkatalysators eine elegante Methode zur Herstellung von 2,7-Octadienol dar. 2,7-Octadienol ist eine wichtige Zwischenstufe zur Synthese von Di-n-octylphthalat, das ein industriell bedeutsamer Weichmacher ist.
Die Synthese verläuft über die Hydrierung von 2,7-Octadienol zu 1-Octanol, das mit Phthalsäureanhydrid zu Di-n-octylphthalat umgesetzt wird (s. Figur I).

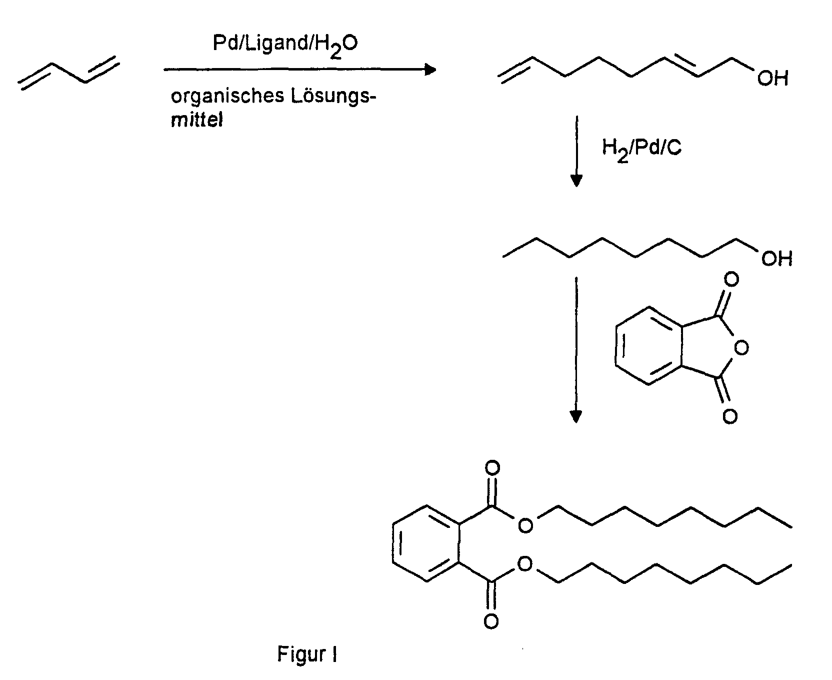

So ist in dem US-Patent Nr. 4,356,333 ein Verfahren zur Telomerisierung von Butadien mit Wasser (Hydrodimerisierung) beschrieben, wobei die Umsetzung des Butadiens mit Wasser in einer wäßrigen Sulfolanlösung in Gegenwart von Palladium oder einer Palladiumverbindung, eines wasserlöslichen einzähnigen Phosphinliganden und einer Aminverbindung mit einer Basenkonstante pKa = 7 oder höher erfolgt.
Aufgrund seiner geringen Kosten und guten Lösungseigenschaften ist als Aminverbindung Triethylamin besonders bevorzugt. Zudem hat sich gezeigt, daß bereits der Austausch von Triethylamin durch Tri-n-propylamin eine Verringerung der Octadienylausbeute zur Folge hat.
Weiter verringert sich bei dem hier beschriebenen Verfahren die Reaktionsgeschwindigkeit beträchtlich, wenn der Gehalt an Sulfolan weniger als 30 Masse-% ist. Übersteigt andererseits der Sulfolangehalt 80 Masse-%, so ist die Extraktionseffizienz von 2,7-Octadienol aus dem Reaktionsgemisch beeinträchtigt. Das vorstehende Verfahren hat den Nachteil, daß die Umsetzung zusätzlich zu einer Aminverbindung ein organisches Lösungsmittel, nämlich Sulfolan, erfordert, wodurch das Verfahren, insbesondere die Produktisolierung, verkompliziert wird.

In dem US Patent Nr. 5,043,487 ist ein Verfahren zur Herstellung von Octadienolen durch Umsetzung von 1,3-Butadien mit Wasser in Gegenwart einer Palladiumverbindung und eines ggf. wasserlöslichen, einzähnigen Phosphinliganden beschrieben, wobei der Zusatz eines Triorganophosphinoxids und von CO₂ wesentlich ist. Zudem erfordert die Reaktion ein organisches Lösungsmittel.

Lösungsmittelfreie Umsetzungen von Butadien und Wasser in Gegenwart einer Palladiumverbindung und eines einzähnigen, wasserlöslichen Phosphinliganden wie z.B. Natriumtrisulfonatophosphin (TPPTS) sind in den US Patenten Nrn. 5,345,007 und 4,142,060 beschrieben.
Gemäß dem US Patent Nr. 5,345,007 erfordert die Reaktion den Zusatz eines tertiären Amines oder einer quartären Ammoniumverbindung mit einem langkettigen Alkylrest, z.B. Dimethyldodecylamin oder Cetyltrimethylammoniumhydroxid, unter einem CO₂-Druck von 10 bar.

Nach der Lehre von GB-A-2 093 025 kann man die Umsetzung von Butadien in Wasser in der Weise modifizieren, indem man dem Reaktionssystem, enthaltend eine Palladiumverbindung und ein einzähniges, wasserlösliches Phosphin, noch zusätzlich ein wasserlösliches, zweizähniges Phosphin hinzugibt. Dabei sollte der Zusatz an wasserlöslichem zweizähnigem Phosphin 3 Mole pro Mole Palladium nicht übersteigen, um nicht einen Abfall in der Reaktionsgeschwindigkeit zu beobachten.

Es ist Aufgabe der vorliegenden Erfindung, ein verbessertes, ökonomisch günstiges Verfahren zur Telomerisierung von Dienen mit einer ein aktives Wasserstoffatom enthaltenden Verbindung zur Verfügung zu stellen, das die entsprechenden Produkte in hoher Ausbeute und Reinheit liefert und das eine einfache Isolierung der Produkte ermöglicht. Insbesondere ist es Aufgabe der Erfindung, ein solches Verfahren zur Verfügung zu stellen, mit dem Butadien in hoher Ausbeute und Selektivität zu 2,7-Octadienol umgesetzt werden kann.

Erfindungsgemäß wird diese Aufgabe durch ein Verfahren gelöst, gemäß dem das Dien mit einer ein aktives Wasserstoffatom enthaltenden Verbindung in Gegenwart einer Palladiumverbindung, eines wasserlöslichen Phosphinliganden, einer Base und Wasser umgesetzt wird, wobei der wasserlösliche Phosphinligand ein zweizähniger Ligand mit der folgenden allgemeinen Formel (I) ist 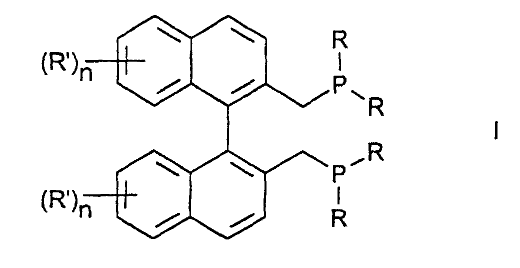 worin
- R: gleich oder verschieden Phenyl, C1-C12-Alkyl und C3-C10-Cycloalkyl, die gegebenenfalls mit einem oder mehreren Resten R' substituiert sein können,
- R': gleich oder verschieden SO₃⁻M⁺,-NMe₃⁺, -COO⁻M⁺
- n: eine ganze Zahl von 1 bis 6, jeweils auf ein Naphthylgerüst bezogen, und
- M: H, Na, K, Cs und R"₄N⁺ mit R" gleich oder verschieden H, C1-C12-Alkyl und C3-C10-Cycloalkyl
bedeuten.

Für das erfindungsgemäße Verfahren besonders geeignete Verbindungen der Formel I sind Verbindungen, worin die Gesamtsumme der R'-Gruppen n^{*} = 2 bis 28, insbesondere n^{*} = 3 bis 10, besonders bevorzugt n^{*} = 4 bis 8, jeweils auf das Gesamtmolekül bezogen, ist, wobei Verbindungen mit R' = M⁺SO₃⁻ ganz besonders bevorzugt sind.

Ein Beispiel für einen besonders bevorzugten Liganden I (BINAS = sulfoniertes II,II'-Bis(diphenylphosphinomethyl)-1,1'-binaphthalin) ist in Figur II dargestellt.

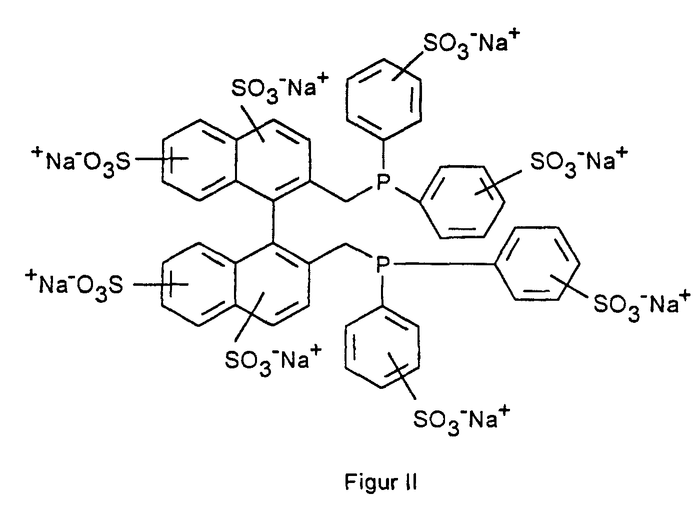

Die erfindungsgemäß eingesetzten zweizähnigen Biphenylphospinliganden I können allgemein durch Sulfonierung des Grundkörpers mit Oleum erhalten werden. Ein Verfahren zur Herstellung ist z.B. in W. A. Herrmann et al., Angew. Chem., 1995, 107, 893, beschrieben.

Das erfindungsgemäße Verfahren erfordert keinen Zusatz von organischen Hilfsstoffen wie die im Stand der Technik eingesetzten Amine oder Triorganophosphinverbindungen. Zudem hat das erfindungsgemäße Verfahren den Vorteil, daß wenn z.B. Wasser als die ein aktives Wasserstoff enthaltende Verbindung eingesetzt wird, die Reaktion kein zusätzliches organisches Lösungsmittel benötigt.

Als Palladiumverbindungen können Pd(0)-Komplexverbindungen und Pd(II)-Verbindungen verwendet werden. Geeignete Beispiele sind Palladiumacetate,-halogenide, -nitrite, -carbonate, -ketonate, -acetylacetonate, Nitrilpalladiumhalogenide, Olefinpalladiumhalogenide, Allylpalladiumhalogenide sowie Palladiumbiscarboxylate.
Konkrete Vertreter sind z.B. Pd(OAc)₂, Pd(acac)₂, (CH₃CN)₂Pd(NO₂)Cl, (C₁₀H₈N₂)PdCl₂, Pd₂(dba)₃ und PdCl₂.

Überraschenderweise wurden auch mit PdCl₂ gute Ergebnisse erzielt, obwohl beschrieben war, daß Palladiumchlorid die Dimerisierung behindert (Tsuji, J. Mori, Y.; Tetrahedron. 1972, 28, 3721).

Der Ligand kann für das erfindungsgemäße Verfahren in üblichen, Fachleuten allgemein bekannten Mengen bezogen auf die Menge Palladiumverbindung eingesetzt werden. Vorzugsweise beträgt das Molverhältnis Ligand zu Palladiumverbindung 1 bis 50, insbesondere 1 bis 10 und ganz besonders bevorzugt 2 bis 6.

Die Palladiumverbindung wird für das erfindungsgemäße Verfahren mit einem Anteil von 10⁻³ - 10 Mol-%, bevorzugt 5x10⁻² - 5 Mol-% und insbesondere bevorzugt 10⁻² - 1 Mol-%, bezogen auf das Dien, eingesetzt.

Für das erfindungsgemäße Verfahren geeignete Diene sind vorzugsweise aliphatische Diene mit 4 bis 8, insbesondere mit 4 bis 6, C-Atomen. Insbesondere bevorzugt sind konjugierte Diene wie Butadien, 1,3-Pentadien und Isopren.

Als Verbindung mit einem aktiven Wasserstoffatom können Wasser, Alkohole ROH mit R = C1 - C8, Phenole, Amine und Säuren wie organische Carbonsäuren verwendet werden.

Beispiele für Alkohole sind primäre, gesättigte oder ungesättigte, geradkettige oder verzweigte aliphatische Alkohole mit vorzugsweise 1 bis 10 Kohlenstoffatomen und die entsprechenden aliphatischen und alicyclischen sekundären Alkohole mit vorzugsweise 3 bis 10 Kohlenstoffatomen. Bevorzugte Beispiele hierfür sind MeOH, EtOH, BuOH, i-PrOH, Cyclohexanol und Allylalkohol.
Beispiele für Amine sind primäre oder sekundäre, aliphatische Alkylamine mit C1 - C8-Alkylketten wie Methylamin, Ethylamin, Dimethylamin und Diethylamin.

Besonders bevorzugt für das erfindungsgemäße Verfahren sind die ein aktives Wasserstoffatom enthaltenden Verbindungen, die bei der Umsetzung, d.h. Telomerisierung, gleichzeitig als Lösungsmittel wirken können, so daß kein zusätzliches, organisches Lösungsmittel erforderlich ist. Beispiele dafür sind insbesondere Wasser und MeOH.

So ist das erfindungsgemäße Verfahren besonders wirkungsvoll für die Telomerisierung von Butadien mit Wasser, da ohne Zusatz von organischen Lösungsmittel und organischen Verbindungen als Hilfsstoffe das industriell wichtige 2,7-Octadienol in hoher Ausbeute und Selektivität auf einfache Weise erhalten werden kann.

Falls erforderlich, kann jedoch der Reaktionsmischung ein geeignetes organisches Lösungsmittel zugesetzt werden, das gegenüber den Bestandteilen der Reaktionsmischung inert ist.
Beispiele hierfür sind Dimethylsulfoxid, Sulfolan, Dimethylformamid, Acetonitril, Aceton, Toluol, Benzonitril oder zB. ein Ether wie Dimethylether, Diethylenglycol und Dimethoxyethan.

Das erfindungsgemäße Verfahren wird in Gegenwart von Basen durchgeführt. Beispiele für bevorzugt verwendete Basen sind die Hydroxide der Alkali- und Erdalkalimetalle sowie Amine, wobei NaOH und KOH aufgrund ihrer leichten Verfügbarkeit besonders bevorzugt sind.

Die Base wird für das erfindungsgemäße Verfahren bevorzugt mit einem Anteil von 0,1 bis 10 Mol-%, besonders bevorzugt 0,3 bis 5 Mol-% und ganz besonders bevorzugt 1-4 Mol-%, bezogen auf das Dien, eingesetzt.

Weiterhin werden für das erfindungsgemäße Verfahren vorteilhaft eine Carbonat- oder/und Hydrogencarbonatverbindung bzw. ein Gemisch davon zugesetzt. Geeignete Beispiele sind die entsprechenden Alkalimetall-bzw. Erdalkalimetallverbindungen.

Die Anteile dieser Carbonat- bzw. Hydrogencarbonatverbindung bzw. von deren Gemischen für das erfindungsgemäße Verfahren sind üblicherweise dieselben wie vorstehend für die Base genannt.

Besonders bevorzugte Vertreter dieser Carbonat- und Hydrogencarbonatverbindungen sind die Natrium- und Kaliumverbindungen.

In einer bevorzugten Ausführungsform wird das erfindungsgemäße Verfahren unter Inertgasatmosphäre durchgeführt, z.B. unter Argon oder Stickstoff.

Zur Durchführung des erfindungsgemäßen Verfahrens können das Dien, eine katalytische Menge der Palladiumverbindung, der zweizähnige Phosphinligand l, die Base, gegebenenfalls mindestens eine Carbonat- oder/und Hydrogencarbonatverbindung sowie gegebenfalls ein organisches Lösungsmittel in einem geeigneten Reaktionsgefäß, z.B. einem Autoklaven, vorzugsweise unter Inertgasatmospäre, vorgelegt werden. Falls erforderlich erfolgt die Vorlage unter Kühlung. Die Umsetzung erfolgt vorzugsweise unter Rühren oder Schütteln.

Das Dien kann auch erst nach Herstellung der Katalysatormischung dem Reaktionssystem zugesetzt werden. Ist das Dien wie im Falle von Butadien gasförmig, kann es in kondensiertem Zustand zugesetzt werden.

Das Reaktionsgemisch wird dann auf die erwünschte Reaktionstemperatur gebracht und vorzugsweise unter Rühren reagieren gelassen. Nach Beendigung der Reaktion wird die Reaktionsmischung, falls erforderlich, auf Umgebungsbedingungen gebracht.

Die Umsetzung erfogt vorzugsweise bei einer Temperatur von 50 bis 100 °C, insbesondere von 70 bis 90 °C. Ist die Temperatur niedriger als 50 °C, verläuft die Reaktion zu langsam; übersteigt die Temperatur 100 °C, können unerwünschte Nebenreaktionen auftreten.

Die Reaktionsführung kann batchweise aber auch kontinuierlich erfolgen.

Das erfindungsgemäße Verfahren kann unter autogenem Reaktionsdruck verlaufen.

Die Aufarbeitung der Reaktionmischung erfolgt über übliche, dem Fachmann geläufige Methoden, z.B. Abdestillieren des überschüssigen Diens und Destillation der Produkte. Der verbleibende Katalysator kann gegebenenfalls wiederverwendet werden. Im Falle zweiphasiger Reaktionsmischungen kann nach beendeter Reaktion die organische Phase (Telomere) von der den Katalysator enthaltenden wässerigen Phase leicht abgetrennt werden.

In einem erfindungsgemäß besonders bevorzugten Verfahren werden die Palladiumverbindung und der zweizähnige Phosphinligand I in einem Gefäß vorgelegt. Dazu werden NaOH und vorzugsweise Na₂CO₃, gelöst in Wasser, gegeben.
In einem weiteren Gefäß wird, gegebenfalls kondensiertes, Butadien bereitgestellt. Die Katalysatormischung wird in einen mit Inertgas gespülten Autoklaven gegeben. Anschließend wird das Butadien zugefügt.
Die Umsetzung erfolgt bei erhöhter Temperatur, vorzugsweise bei 70 - 90 °C, über mehrere Stunden, vorzugsweise 2 -4 Stunden.
Anschließend wird die Reaktionsmischung auf Raumtemperatur oder etwas darüber abgekühlt und das nicht-umgesetzte Butadien in ein auf -78°C gekühltes Gefäß überführt.
Von der auf Raumtemperatur gekühlten Reaktionsmischung wird die organische Phase abgetrennt und das gewünschte Produkt daraus nach einer herkömmlichen Methode wie z.B. Destillation isoliert.

Nach diesem Verfahren kann 2,7-Octadienol in hoher Ausbeute und guter Selektivität auf einfache Weise ohne Zusatz weiterer Lösungsmittel oder Hilfsstoffe gewonnen werden.

Das erfindungsgemäße Verfahren wird nachfolgend anhand illustrativer Beispiele näher erläutert ohne es jedoch darauf zu begrenzen.

### BEISPIELE 1 bis 5:

In einen Schlenkapparat wurde Pd(OAc)₂ (336,8 mg, 1,5 mmol), das in DMSO (1,7 ml, 23,7 mmol) gelöst war, gegeben. Zu der Lösung wurde BINAS (43,6 mg, 6 mmol) gegeben, wobei eine exotherme Reaktion (30 °C) beobachtet wurde. Dieser Mischung wurde Na₂CO₃ (795 mg, 7,5 mmol) und NaOH (900mg, 22,5 mmol) zugesetzt, die in H₂O (18 ml, 1000 mmol) gelöst waren, und die erhaltene Mischung wurde auf -15 °C gekühlt. In einen anderen Schlenkapparat, der auf -78 °C abgekühlt worden war, wurde Butadien (714 mmol) gegeben.
Ein 250 ml Autoklav wurde auf -78 °C abgekühlt und mit N₂ gespült.
Unter N₂ wurden zu dem Autoklaven die Katalysatormischung sowie anschließend das Butadien gegeben. Der Autoklav wurde geschlossen, zunächst auf Raumtemperatur und anschließend auf 90 °C erwärmt.
Nach 4 Stunden unter Rühren bei 90 °C wurde die Reaktionsmischung auf 50 °C abgekühlt und das nicht-umgesetzte Butadien über ein Ventil in einen auf - 78 °C gekühlten Schlenkapparat überführt, um das nicht-umgesetzte Butadien wiederzugewinnen.

Der Autoklav wurde nun auf Raumtemperatur gekühlt und entleert. Die erhaltene organische Phase wurde abgetrennt und gaschromatographisch untersucht.
Diese Reaktion wurde auf gleiche Weise für verschiedene Palladiumverbindungen durchgeführt.

Dabei wurden die nachstehend in der Tabelle aufgeführten Ergebnisse erhalten:

| Beispiel | Pd-Verbindung | 2,7-Octadienol | 1,7-Octadien-3-ol | Vinylcyclohexen + 1,3,7-Octatrien |
|---|---|---|---|---|
| 1 | Pd(OAc)₂ | 64,1 | 13,3 | 7,0 |
| | | | | |
| 2 | Pd(acac)₂ | 63,9 | 11,2 | 6,4 |
| | | | | |
| 3 | (CH₃CN)₂Pd(NO₂)Cl | 63,5 | 12,1 | 7,6 |
| | | | | |
| 4 | (C₁₀H₈N₂)PdCl₂ | 59,2 | 13,9 | 8,5 |
| | | | | |
| 5 | PdCl₂ | 64,9 | 10,0 | 7,2 |
| (Angaben zur Ausbeute jeweils in Gew.-%) | | | | |

Bei den Resten handelte es sich um nicht näher identifizierte Verbindungen.

### BEISPIEL 6 und VERGLEICHSBEISPIEL:

Es wurde wie in Beispielen 1 bis 5 verfahren, wobei als Pd-verbindung Pd(OAc)₂ und als Ligand BINAS bzw. Natriumtrisulfonatophosphin (TPPTS), ein einzähniger Ligand, verwendet wurden. Die Reaktionsbedingungen waren 80 °C und 16 Stunden. Die erhaltene organische Phase wurde gaschromatographisch untersucht, wobei die nachfolgend aufgeführten Ergebnisse erhalten wurden:

| | 2,7-Octadienol | 1,7-Octadien-3-ol | Vinylcyclohexen + 1,3,7-Octatrien | |
|---|---|---|---|---|
| BINAS | 70 % | 12 % | 9 % | (B6) |
| TPPTS | 46 % | 14 % | 11 % | (VB) |
| (Angaben zur Ausbeute jeweils in Gew.-%) | | | | |

Bei den Resten handelte es sich um nicht näher identifizierte Verbindungen.

Alle erfindungsgemäßen Beispiele und das Vergleichsbeispiel ergaben aufgrund dessen, daß Wasser sowohl als eine Verbindung mit einem aktiven Wasserstoffatom, als auch als Lösungsmittel eingesetzt wurde, eine zweiphasige Reaktionsmischung, bei der die organische Phase (Telomere) von der wässerigen Katalysatorphase getrennt vorlag und sich daher leicht abtrennen ließ.

## Patentansprüche

1. Verfahren zur Telomerisierung von Dienen, wobei das Dien mit einer ein aktives Wasserstoffatom enthaltenden Verbindung in Gegenwart einer Palladiumverbindung, eines wasserlöslichen Phosphinliganden, einer Base und Wasser umgesetzt wird, dadurch gekennzeichnet, daß der wasserlösliche Phosphinligand ein zweizähniger Ligand mit der allgemeinen Formel I 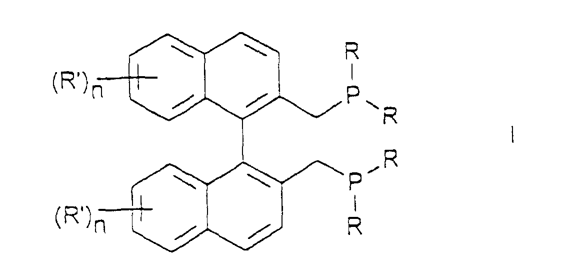 ist, worin
R gleich oder verschieden Phenyl, C1-C12-Alkyl und C3-C10-Cycloalkyl, die gegebenenfalls mit einem oder mehreren Resten R' substituiert sein können,
R' gleich oder verschieden SO₃⁻M⁺, -NMe₃⁺, -COO⁻M⁺
n eine ganze Zahl von 1 bis 6, jeweils auf ein Naphthylgerüst bezogen, und
M H, Na, K, Cs und R"₄N⁺ mit R" gleich oder verschieden H, C1-C12-Alkyl und C3-C10-Cycloalkyl
bedeuten.

2. Verfahren nach Anspruch 1 wobei die Gesamtsumme der R'-Gruppen n^{*}=2 bis 28, insbesondere n^{*}= 3 bis 10, jeweils auf das Gesamtmolekül bezogen, ist.

3. Verfahren nach Anspruch 1, wobei der zweizähnige Phosphinligand I ein Ligand mit der folgenden Formel ist

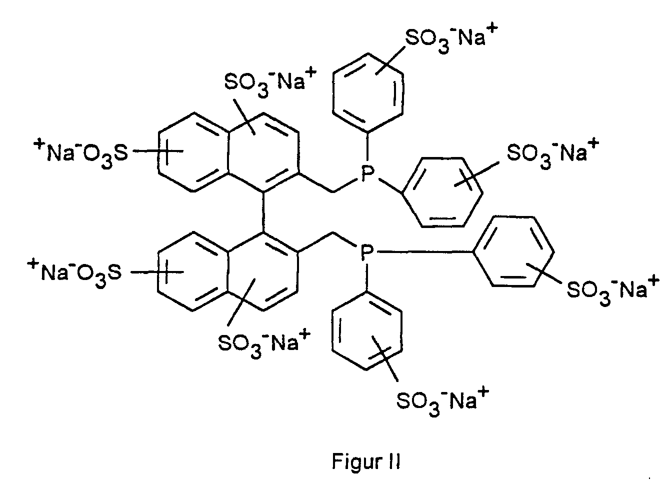

4. Verfahren nach einem der vorhergehenden Ansprüche, wobei die Palladiumverbindung ausgewählt wird unter Palladiumacetaten, -halogeniden, -nitriten, -carbonaten, -ketonaten, -acetylketonaten, Nitrilpalladiumhalogeniden, Olefinpalladiumhalogeniden, Allylpalladiumhalogeniden und Palladiumbiscarboxylaten.

5. Verfahren nach Anspruch 4, wobei die Palladiumverbindung ausgewählt wird unter Pd(OAc)₂, Pd(acac)₂, (CH₃CN)₂Pd(NO₂)Cl, (C₁₀H₈N₂)PdCl₂, Pd₂(dba)₃ und PdCl₂.

6. Verfahren nach einem der vorhergehenden Ansprüche, wobei das Dien ausgewählt wird unter aliphatischen Dienen mit 4 bis 8 Kohlenstoffatomen.

7. Verfahren nach Anspruch 6, wobei das Dien ein konjugiertes Dien ist.

8. Verfahren nach Anspruch 7, wobei das Dien ausgewählt wird unter Butadien, 1,3-Pentadien und Isopren.

9. Verfahren nach einem der vorhergehenden Ansprüche, wobei die ein aktives Wasserstoffatom enthaltende Verbindung ausgewählt wird unter Wasser, C1-C8-Alkoholen, Phenolen, Aminen und Säuren.

10. Verfahren nach Anspruch 9, wobei die ein aktives Wasserstoffatom enthaltende Verbindung ausgewählt wird unter Wasser und MeOH.

11. Verfahren nach einem der vorhergehenden Ansprüche, wobei die Base ausgewählt wird unter einem Alkalimetallhydroxid, Erdalkalimetallhydroxid und einem Amin.

12. Verfahren nach Anspruch 11, wobei die Base NaOH oder KOH ist.

13. Verfahren nach Anspruch 11 oder 12, wobei die Base in einem Anteil von 0,1 bis 10 Mol%, bezogen auf das Dien, eingesetzt wird.

14. Verfahren nach einem der vorhergehenden Ansprüche, wobei dem Reaktionsgemisch zusätzlich mindestens eine Verbindung ausgewählt unter Carbonat- und Hydrogencarbonatverbindungen und deren Gemischen zugesetzt wird.

15. Verfahren nach Anspruch 14, wobei die Verbindung ein Natrium- oder Kaliumsalz ist.

16. Verfahren nach Anspruch 14 oder 15, wobei die Verbindung in einem Anteil von 0,1 bis 10 Mol%, bezogen auf das Dien, zugesetzt wird.

17. Verfahren nach einem der vorhergehenden Ansprüche, wobei die Umsetzung bei einer Temperatur ausgewählt aus dem Bereich von 50 °C bis 100°C erfolgt.

18. Verfahren nach Anspruch 17, wobei die Temperatur aus einem Bereich von 70 °C bis 90°C ausgewählt wird.

19. Verfahren nach einem der vorhergehenden Ansprüche, wobei das Verfahren unter Inertgasatmosphäre durchgeführt wird.

20. Verfahren zur Herstellung von Di-n-octylphthalat, wobei zunächst 2,7-Octadienol nach einem Verfahren gemäß einem der vorhergehenden Ansprüche erzeugt wird, indem als Dien Butadien und als eine ein aktives Wasserstoffatom enthaltenden Verbindung Wasser eingesetzt werden, das erhaltene 2,7-Octadienol zu 1-Octanol hydriert und anschließend das erhaltene 1-Octanol mit Phthalsäureanhydrid zu Di-n-octylphthalat umgesetzt wird.

## Claims

1. A process for the telomerization of dienes, the diene being reacted with a compound containing an active hydrogen atom in the presence of a palladium compound, a water-soluble phosphine ligand, a base and water, wherein the water-soluble phosphine ligand is a bidentate ligand having the formula I 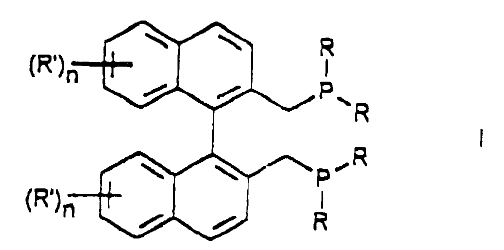 in which
R is identical or different and is phenyl, C₁-C₁₂-alkyl or C₃-C₁₀-cycloalkyl, which may be unsubstituted or substituted by one or more radicals R',
R' is identical or different and is SO₃⁻M⁺, -NMe₃⁺ or -COO⁻M⁺,
n is an integer from 1 to 6, in each case based on a naphthyl backbone, and
M is H, Na, K, Cs or R"₄N⁺ where R" is identical or different and is H, C₁-C₁₂-alkyl or C₃-C₁₀-cycloalkyl.

2. The process as claimed in claim 1, where the total number of R' groups is n^{*} = from 2 to 28, in particular n^{*} = from 3 to 10, in each case based on the overall molecule.

3. The process as claimed in claim 1, where the bidentate phosphine ligand I is a ligand having the following formula

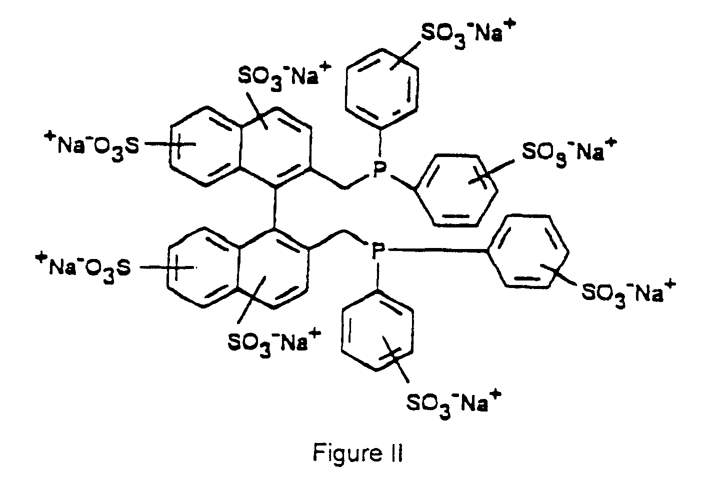

4. The process as claimed in one of the preceding claims, where the palladium compound is chosen from palladium acetates, halides, nitrites, carbonates, ketonates, acetylketonates, nitrile palladium halides, olefinpalladium halides, allylpalladium halides and palladium biscarboxylates.

5. The process as claimed in claim 4, where the palladium compound is chosen from Pd(OAc)₂, Pd(acac)₂, (CH₃CN)₂Pd(NO₂)Cl, (C₁₀H₈N₂)PdCl₂, Pd₂(dba)₃ and PdCl₂.

6. The process as claimed in one of the preceding claims, where the diene is chosen from aliphatic dienes having from 4 to 8 carbon atoms.

7. The process as claimed in claim 6, where the diene is a conjugated diene.

8. The process as claimed in claim 7, where the diene is chosen from butadiene, 1,3-pentadiene and isoprene.

9. The process as claimed in one of the preceding claims, where the compound containing an active hydrogen atom is chosen from water, C₁-C₈-alcohols, phenols, amines and acids.

10. The process as claimed in claim 9, where the compound containing an active hydrogen atom is chosen from water and MeOH.

11. The process as claimed in one of the preceding claims, where the base is chosen from an alkali metal hydroxide, alkaline earth metal hydroxide and an amine.

12. The process as claimed in claim 11, where the base is NaOH or KOH.

13. The process as claimed in claim 11 or 12, where the base is used in an amount of from 0.1 to 10 mol%, based on the diene.

14. The process as claimed in one of the preceding claims, where at least one compound chosen from carbonate and hydrogencarbonate compounds and mixtures thereof is additionally added to the reaction mixture.

15. The process as claimed in claim 14, where the compound is a sodium or potassium salt.

16. The process as claimed in claim 14 or 15, where the compound is added in an amount of from 0.1 to 10 mol%, based on the diene.

17. The process as claimed in one of the preceding claims, where the reaction takes place at a temperature chosen from the range from 50 °C to 100 °C.

18. The process as claimed in claim 17, where the temperature is chosen from a range from 70 °C to 90 °C.

19. The process as claimed in one of the preceding claims, where the process is carried out under an inert-gas atmosphere.

20. A process for the preparation of di-n-octyl phthalate, which comprises firstly producing 2,7-octadienol by a process as claimed in one of the preceding claims in which the diene used is butadiene and the compound containing an active hydrogen atom is water, hydrogenating the resulting 2,7-octadienol to give 1-octanol and subsequently reacting the resulting 1-octanol with phthalic anhydride to give di-n-octyl phthalate.

## Revendications

1. Procédé de télomérisation de diènes où le diène est mis à réagir avec un composé contenant un atome d'hydrogène actif en présence d'un composé du palladium, d'un ligand phosphine hydrosoluble, d'une base et d'eau, caractérisé en ce que le ligand phosphine hydrosoluble est un ligand bidenté ayant la formule générale I 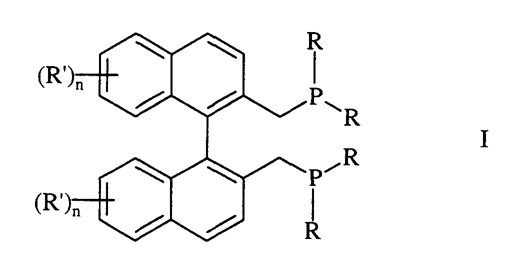 où
R représente phényle, alkyle en C₁-C₁₂ et cycloalkyle en C₃-C₁₀ identiques ou différents qui peuvent éventuellement être substitués par un ou plusieurs restes R',
R' représente SO₃⁻M⁺, -NMe₃⁺, -COO⁻M⁺ identiques ou différents
n représente un nombre entier de 1 à 6, à chaque fois rapporté à un squelette naphtyle, et
M représente H, Na, K, Cs et R"₄N⁺ où R" représente H, alkyle en C₁-C₁₂ et cycloalkyle en C₃-C₁₀ identiques ou différents.

2. Procédé selon la revendication 1, où la somme totale des groupes R' n^{*} = 2 à 28, en particulier n^{*} = 3 à 10, dans chaque cas rapportée à la molécule totale.

3. Procédé selon la revendication 1, où le ligand phosphine bidenté I est un ligand ayant la formule suivante

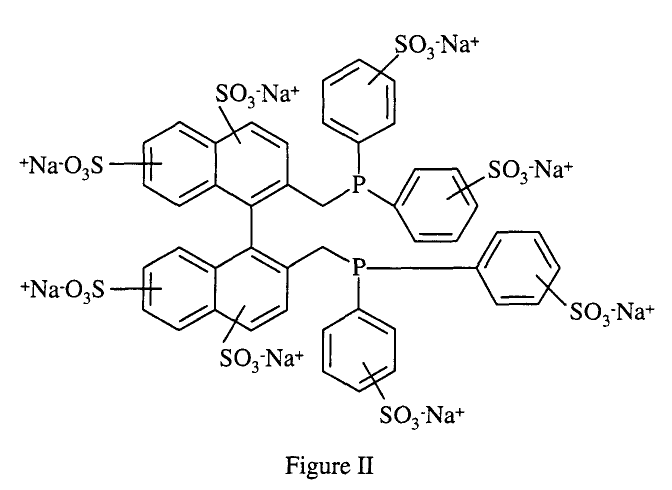

4. Procédé selon l'une des revendications précédentes, où le composé du palladium est choisi parmi les acétates, halogénures, nitrures, carbonates, cétonates, acétylcétonates de palladium, les halogénures de nitrilepalladium, les halogénures d'oléfinepalladium, les halogénures d'allylpalladium et les biscarboxylates de palladium.

5. Procédé selon la revendication 4, où le composé du palladium est choisi parmi Pd(OAc)₂, Pd(acac)₂, (CH₃CN)₂Pd(NO₂)Cl, (C₁₀H₈N₂)PdCl₂, Pd₂(dba)₃ et PdCl₂.

6. Procédé selon l'une des revendications précédentes, où le diène est choisi parmi les diènes aliphatiques ayant 4 à 8 atomes de carbone.

7. Procédé selon la revendication 6, où le diène est un diène conjugué.

8. Procédé selon la revendication 7, où le diène est choisi parmi le butadiène, le 1,3-pentadiène et l'isoprène.

9. Procédé selon l'une des revendications précédentes, où le composé contenant un atome d'hydrogène actif est choisi parmi l'eau, les alcools en C₁-C₈, les phénols, les amines et les acides.

10. Procédé selon la revendication 9, où le composé contenant un atome d'hydrogène actif est choisi parmi l'eau et MeOH.

11. Procédé selon l'une des revendications précédentes, où la base est choisie parmi un hydroxyde de métal alcalin, un hydroxyde de métal alcalino-terreux et une amine.

12. Procédé selon la revendication 11, où la base est NaOH ou KOH.

13. Procédé selon la revendication 11 ou 12, où la base est utilisée en une proportion de 0,1 à 10 mol %, par rapport au diène.

14. Procédé selon l'une des revendications précédentes, où au moins un composé choisi parmi les composés de carbonate et hydrogénocarbonate et leurs mélanges est ajouté en outre au mélange réactionnel.

15. Procédé selon la revendication 14, où le composé est un sel de sodium ou de potassium.

16. Procédé selon la revendication 14 ou 15, où le composé est ajouté en une proportion de 0,1 à 10 mol % par rapport au diène.

17. Procédé selon l'une des revendications précédentes, où la réaction a lieu à une température choisie dans le domaine de 50°C à 100°C.

18. Procédé selon la revendication 17, où la température est choisie dans un domaine de 70°C à 90°C.

19. Procédé selon l'une des revendications précédentes, où le procédé est mis en oeuvre sous une atmosphère de gaz inerte.

20. Procédé de préparation du phtalate de di-n-octyle où, tout d'abord, le 2,7-octadiénol est produit selon un procédé selon l'une des revendications précédentes, par le fait que le butadiène est utilisé comme diène et l'eau est utilisée comme composé contenant un atome d'hydrogène actif, le 2,7-octadiénol obtenu est hydrogéné en 1-octanol puis le 1-octanol obtenu est converti en phtalate de di-n-octyle avec l'anhydride phtalique.
